# EUROPEAN PATENT APPLICATION

(11) **EP 4 523 742 A1**
(43) Date of publication of application: **19.03.2025**
(21) Application number: 22944633.1
(22) Date of filing: 29.11.2022
(51) Int. Cl.: A61N 7/00

(54) **CONTROL METHOD FOR OUTPUTTING ULTRASONIC ENERGY, CONTROL HOST, SYSTEM, AND TREATMENT DEVICE**

(30) Priority: 02.06.2022 CN 202210623467
(71) Applicant: Shenzhen Peninsula Medical Group, Shenzhen, Guangdong 518000 (CN)
(72) Inventor: LI, Yanan, Shenzhen, Guangdong 518000 (CN); LEI, Xiaobing, Shenzhen, Guangdong 518000 (CN); LI, Xingli, Shenzhen, Guangdong 518000 (CN); PENG, Yujia, Shenzhen, Guangdong 518000 (CN)
(74) Representative: Osterhoff, Utz
(86) International application number: PCT/CN2022/134882
(87) International publication number: WO 2023/231332

(57) **Abstract**

Disclosed are a control method for outputting ultrasound energy, a control host, a system, and a treatment device. The control method for outputting the ultrasound energy includes: step S10, detecting a moving speed of an ultrasound treatment applicator; and step S20, controlling a transducer to output the ultrasound energy according to the detected moving speed; and when the detected moving speed is less than or equal to a first preset speed value, the transducer is controlled to stop outputting the ultrasound energy.

## Description

The present application claims priority to Chinese Patent Application No. 202210623467.6, filed on June 2, 2022 and entitled "CONTROL METHOD FOR OUTPUTTING ULTRASOUND ENERGY, CONTROL HOST, SYSTEM AND TREATMENT DEVICE". The disclosures of the above-mentioned applications are incorporated herein by reference in their entireties.

### TECHNICAL FIELD

The present application relates to the technical field of ultrasound treatment equipment, in particular to a control method for outputting ultrasound energy, a control host, a system, and a treatment device.

### BACKGROUND

Ultrasonic treatment is a common treatment means, which achieves therapeutic effect, with weight loss and wrinkle removal through the output of the ultrasonic energy to the subcutaneous tissue.

In the process of the ultrasound treatment, the ultrasound energy acts on the subcutaneous tissue through the skin. During the operation, the position of the ultrasound energy output needs to be constantly changed, and the area of the ultrasound energy output can be diffused to achieve the therapeutic effect.

### SUMMARY

When the operator operates, due to improper personal operation, the output of ultrasound energy to the treatment area cannot be well controlled, and the expected therapeutic effect cannot be achieved. For example, multiple outputs of ultrasound energy at the same location on the skin can cause the energy in that location to be too high, which can have the side effect of burning the skin.

The main purpose of the present application is to provide a control method for outputting ultrasound energy, a control host, a system, and a treatment device, aiming at solving the problem that ultrasound treatment cannot achieve the expected therapeutic effect due to improper personal operation.

In order to achieve the above purpose, the present application provides a control method for outputting ultrasound energy, including the following steps:
step S10, detecting a moving speed of an ultrasound treatment applicator; and
step S20, controlling a transducer to output the ultrasound energy according to the detected moving speed,
wherein in response to that the detected moving speed is less than or equal to a first preset speed value, the transducer is controlled to stop outputting the ultrasound energy.

In an embodiment, the step S20 further includes the following step:
in response to that the detected moving speed is greater than the first preset speed value and smaller than a second preset speed value, controlling the ultrasound energy output by the transducer to increase according to an increase of the detected moving speed.

In an embodiment, the step S20 further includes the following step:
in response to that the detected moving speed is greater than the first preset speed value and smaller than a second preset speed value, controlling the transducer to output a first preset ultrasound energy.

In an embodiment, the step S20 further includes the following step:
in response to that the detected moving speed is greater than or equal to the second preset speed value and smaller than a third preset speed value, controlling the transducer to output a second preset ultrasound energy.

In an embodiment, the step S20 further includes the following step:
in response to that the detected moving speed is greater than or equal to a third preset speed value, controlling the transducer to stop outputting the ultrasound energy and output a corresponding alarm signal.

In an embodiment, in response to that the detected moving speed is greater than the first preset speed value and smaller than the second preset speed value, the ultrasound energy output by the transducer is controlled to increase linearly according to the increase of the detected moving speed, that is, a growth rate of the output ultrasound energy with respect to the moving speed remains unchanged.

In an embodiment, in response to that the detected moving speed is greater than the first preset speed value and smaller than the second preset speed value, the ultrasound energy output by the transducer is controlled to increase exponentially according to the increase of the detected moving speed, that is, a growth rate of the output ultrasound energy with respect to the moving speed increases according to the increase of the detected moving speed.

In an embodiment, in response to that the detected moving speed is greater than the first preset speed value and smaller than the second preset speed value, the ultrasound energy output by the transducer is controlled to increase logarithmically according to the increase of the detected moving speed, that is, a growth rate of the output ultrasound energy with respect to the moving speed decreases according to the increase of the detected moving speed.

In an embodiment, in response to that the detected moving speed is greater than the first preset speed value and smaller than the second preset speed value, controlling the ultrasound energy output by the transducer to increase linearly in segments according to the increase of the detected moving speed includes the following steps:
detecting a preset speed interval divided from the moving speed greater than the first preset speed value and smaller than the second preset speed value;
determining a growth rate of the output ultrasound energy with respect to the moving speed according to the detected preset speed interval; and
controlling the transducer to adjust the output ultrasound energy according to the determined growth rate and the change of the moving speed.

In an embodiment, before the step S10, the control method further includes the following steps:
detecting whether a surface of the ultrasound treatment applicator is in sufficient contact with a skin; and
in response to that the surface of the ultrasound treatment applicator is in sufficient contact with the skin, performing the step S10; or, in response to that the surface of the ultrasound treatment applicator is in insufficient contact with the skin, outputting a corresponding alarm signal.

In an embodiment, the step S20 further includes the following steps:
detecting whether the surface of the ultrasound treatment applicator is in sufficient contact with the skin; and
in response to that the surface of the ultrasound treatment applicator is in insufficient contact with the skin, controlling the transducer to stop outputting the ultrasound energy and output the corresponding alarm signal; or, in response to that the surface of the ultrasound treatment applicator is in sufficient contact with the skin, performing the step S10.

In an embodiment, the step S20 further includes the following steps:
detecting a temperature of a skin surface; and
in response to that the temperature on the surface of the ultrasound treatment applicator is higher than a preset temperature value, controlling the transducer to stop outputting the ultrasound energy and output a corresponding alarm signal; or, in response to that the temperature on the surface of the ultrasound treatment applicator is lower than the preset temperature value, outputting an operation guide signal, and controlling the transducer to continue to output the ultrasound energy.

In an embodiment, the control method for outputting the ultrasound energy further includes the following step: controlling an ultrasound output power and/or an ultrasound pulse output repetition frequency to control an ultrasound output energy of the transducer.

In an embodiment, the control method for outputting the ultrasound energy further includes the following step: controlling a time and/or a voltage amplitude of an output pulse of an ultrasound unit to control the ultrasound output power of the transducer.

In an embodiment, the control method for outputting the ultrasound energy further includes the following step: controlling a frequency of the output pulse of the ultrasound unit and/or a duty ratio of the output pulse of the ultrasound unit to control the time of the output pulse of the ultrasound unit.

The present application provides a control host, including a memory and a processor; a control program for outputting ultrasound energy is stored in the memory, and when the control program for outputting the ultrasound energy is performed by the processor, the steps of the control method for outputting the ultrasound energy are realized.

The present application provides an ultrasound treatment system, including:
an ultrasound treatment applicator provided with a transducer inside;
an ultrasound handle unit; wherein the ultrasound treatment applicator is mounted on the ultrasound handle unit, and the ultrasound handle unit is configured to output ultrasound energy when sliding on a skin surface;
a sensor assembly arranged on the ultrasound treatment applicator or the ultrasound handle unit and configured for detecting a moving speed of the ultrasound treatment applicator;
an alarm configured for alarming according to the detected moving speed;
the control host; and the control host is connected with the ultrasound handle unit and configured to control the operation of the ultrasound treatment applicator.

In an embodiment, the sensor assembly is also configured to detect the temperature of the skin surface and/or whether the skin surface is in sufficient contact with the surface of the ultrasound treatment applicator; and the alarm is also configured to detect the temperature of the skin surface and/or alarm based on whether the skin surface is in sufficient contact with the surface of the ultrasound treatment applicator.

In an embodiment, the control host further includes a system control unit and an ultrasound drive unit; the system control unit configures working parameters of the ultrasound treatment applicator, and the ultrasound drive unit is configured to drive the ultrasound treatment applicator to work based on the operating parameters configured by the system control unit;
the system control unit is configured to configure the ultrasound output power and the ultrasound pulse output repetition frequency, and the ultrasound drive unit is configured to drive the ultrasound treatment applicator to work according to the ultrasound output power and the ultrasound pulse output repetition frequency configured by the system control unit.

In an embodiment, the system control unit is further configured to configure the time and/or the voltage amplitude of the output pulse of the ultrasound unit to configure the ultrasound output power; and the ultrasound drive unit is configured to drive the ultrasound treatment applicator to work based on the ultrasound output power and ultrasound pulse output repetition frequency configured by the system control unit.

The present application provides an ultrasound treatment device, and the ultrasound treatment device includes the ultrasound treatment system.

The present application uses a speed measuring sensor and an ultrasound treatment transducer. The speed measuring sensor is configured to detect the moving speed of the control method for outputting the ultrasound energy, and the ultrasound treatment transducer is configured to output the ultrasound energy according to the moving speed detected by the speed measuring sensor. When the moving speed detected by the speed measuring sensor is less than or equal to the preset speed value, the ultrasound treatment transducer stops outputting the ultrasound energy to the subcutaneous tissue. When working, the speed measuring sensor detects the possibility of the sliding treatment applicator stopping on the skin surface by detecting the moving speed of the control method for outputting the ultrasound energy. When the speed detected by the speed measuring sensor is less than or equal to the preset speed value, it is determined that the control method for outputting the ultrasound energy is small enough or the control method for outputting the ultrasound energy is stopped, and the ultrasound treatment transducer stops outputting the ultrasound energy to the subcutaneous tissue to avoid the possibility of multiple output of ultrasound energy at the same position of the skin, and reduce the risk of scalding the skin. The present application solves the problem of scalding the skin configured by multiple output of ultrasound energy at the same position of the skin by setting such a control method for outputting the ultrasound energy.

### BRIEF DESCRIPTION OF THE DRAWINGS

In order to more clearly illustrate the embodiments of the present application or the technical solutions in the related art, the accompanying drawings that need to be configured in the description of the embodiments or the related art will be briefly introduced below. Obviously, the accompanying drawings in the following description are only some embodiments of the present application, and those skilled in the art can also obtain other drawings according to the structures shown in these drawings without creative effort.
FIG. 1 is a working flow chart when the detected moving speed is less than or equal to the first preset speed value in an embodiment of the control method for outputting the ultrasound energy in the present application.
FIG. 2 is a working flow chart when the detected moving speed is greater than the first preset speed value and less than the second preset speed value in an embodiment of the control method for outputting the ultrasound energy in the present application.
FIG. 3 is a working flow chart when the detected moving speed is greater than or equal to the second preset speed value in an embodiment of the control method for outputting the ultrasound energy in the present application.
FIG. 4 is a working flow chart when the detected moving speed is greater than or equal to the third preset speed value in an embodiment of the control method for outputting the ultrasound energy in the present application.
FIG. 5 is a linear change relationship diagram of the ultrasound output energy of the transducer with respect to the moving speed in an embodiment of the control method for outputting the ultrasound energy in the present application.
FIG. 6 is a linear change relationship diagram of the ultrasound output energy of the transducer with respect to the moving speed in an embodiment of the control method for outputting the ultrasound energy in the present application.
FIG. 7 is an exponential change relationship diagram of the ultrasound output energy of the transducer with respect to the moving speed in an embodiment of the control method for outputting the ultrasound energy in the present application.
FIG. 8 is a logarithmic change relationship diagram of the ultrasound output energy of the transducer with respect to the moving speed in an embodiment of the control method for outputting the ultrasound energy in the present application.
FIG. 9 is a segmental change relationship diagram of the ultrasound output energy of the transducer with respect to the moving speed in an embodiment of the control method for outputting the ultrasound energy in the present application.
FIG. 10 is a schematic diagram of output pulse modulation in an embodiment of the ultrasound energy control method of the present application.
FIG. 11 is a schematic structural diagram in an embodiment of the ultrasound treatment system of the present application.

The realization of the purpose of the present application, functional characteristics and advantages will be further described with reference to the accompanying drawings in conjunction with the embodiments.

### DETAILED DESCRIPTION OF THE EMBODIMENTS

The following will clearly and completely describe the technical solutions in the embodiments of the present application with reference to the accompanying drawings in the embodiments of the present application. Obviously, the described embodiments are only part of the embodiments of the present application, not all of them. Based on the embodiments in the present application, all other embodiments obtained by those skilled in the art without creative efforts fall within the protection scope of the present application. It should be noted that if there are directional indications (such as up, down, left, right, front, back...) in the embodiment of the present application, the directional indications are only configured to explain the relative positional relationship and movement of each part in a specific posture (as shown in the accompanying drawings). If the specific posture changes, the directional indications will also change accordingly.

In addition, if there are descriptions involving "first", "second", etc. in the embodiments of the present application, the descriptions of "first", "second", etc. are only for descriptive purposes, and should not be interpreted as indications or hints its relative importance or implicit indication of the number of technical features indicated. Thus, the features defined as "first" and "second" may explicitly or implicitly include at least one of these features. In addition, the technical solutions of the various embodiments can be combined with each other, but it must be based on the realization of those skilled in the art. When the combination of technical solutions is contradictory or cannot be realized, it should be considered that the combination of technical solutions does not exist, nor within the protection scope required by the present application.

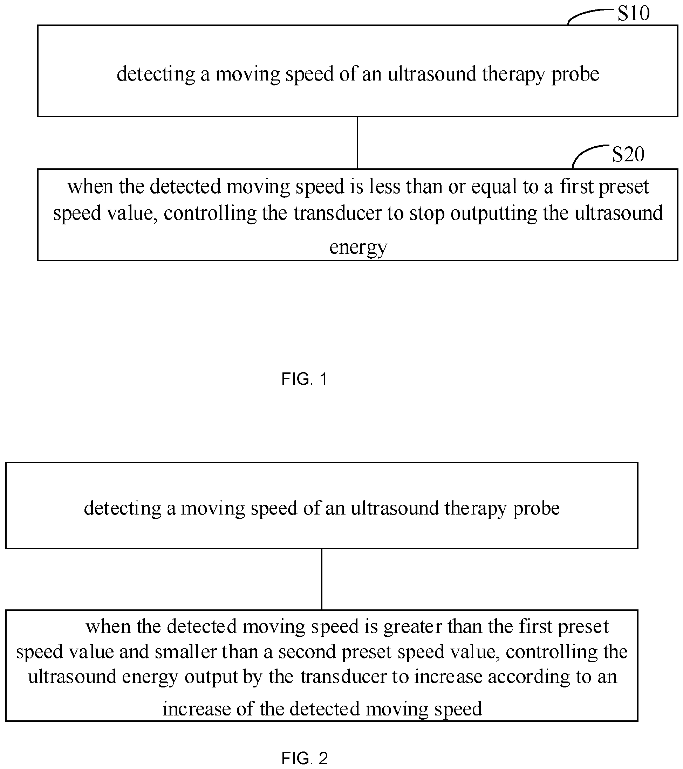

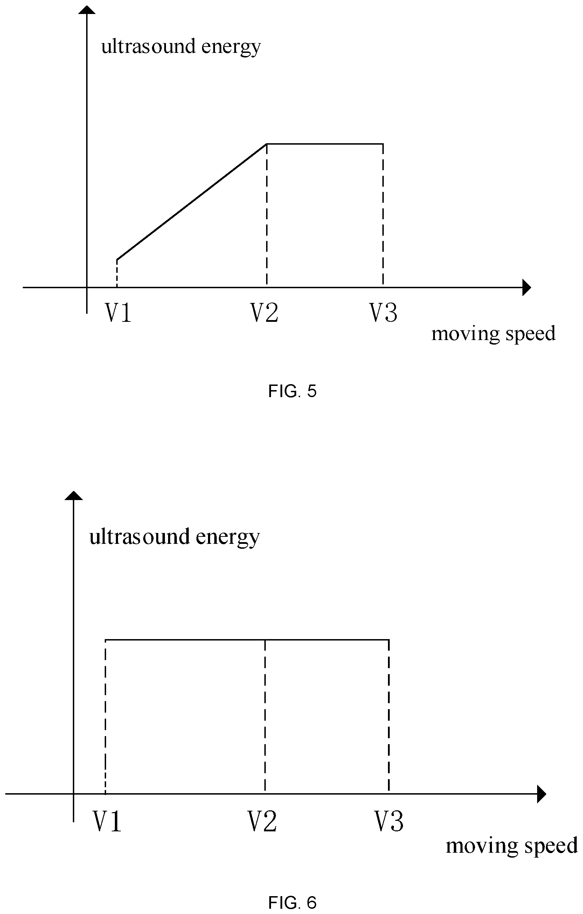

Referring to FIG. 1 and FIG. 5, in an embodiment of the present application, the control method for outputting the ultrasound energy includes the following steps:
Step S10, detecting the moving speed of the ultrasound treatment applicator;
Step S20, controlling the transducer to output the ultrasound energy according to the detected moving speed; when the detected moving speed is less than or equal to the first preset speed value, the transducer is controlled to stop outputting the ultrasound energy.

The ultrasound treatment transducer achieves the therapeutic effect by outputting the ultrasound energy to the subcutaneous tissue, and the treatment applicator is slid continuously during the treatment. However, when the existing treatment applicator is improperly operated by the operator, the sliding treatment applicator may stop on the skin surface, and the output of ultrasound energy to the treatment area cannot be controlled well, and the expected therapeutic effect cannot be achieved.

During ultrasound treatment, the output of ultrasound energy in the operating area is controlled to achieve therapeutic effects. There is a matching relationship between the moving speed of the ultrasound treatment applicator and the output of ultrasound energy, that is, the ultrasound energy output is set according to the moving speed of the ultrasound treatment applicator to achieve the desired therapeutic effect.

In this embodiment, the moving speed of the ultrasound treatment applicator is detected, and the ultrasound energy output by the transducer is controlled according to the detected moving speed. The control of ultrasound energy is realized based on the detected moving speed. The moving speed of the ultrasound treatment applicator is divided into multiple preset speed values, and multiple preset speed values form multiple preset speed intervals. There is a control logic for ultrasound energy output in each preset speed interval. Since the control logic corresponding to each preset speed interval is set based on improving the effect of ultrasound treatment, during operation, using the control logic to control the output of ultrasound energy can make the ultrasound treatment achieve the desired effect.

During operation, the moving speed of the ultrasound treatment applicator is detected to obtain the preset speed range of the moving speed of the ultrasound treatment applicator, and the ultrasound energy output is controlled according to the control logic of the preset speed range.

For example, because the ultrasound energy is output at the same position of the skin multiple times, the energy at this position will be too high, which will not only fail to produce the expected therapeutic effect, but also cause the side effect of scalding the skin. Therefore, when the ultrasound treatment applicator does not move or moves at a low speed, the transducer should not output ultrasound energy.

When the detected speed is less than or equal to the first preset speed value, it is determined that the speed of the ultrasound treatment applicator is small enough or the ultrasound treatment applicator stops moving. If the ultrasound treatment transducer outputs ultrasound energy to the subcutaneous tissue at this time, there is a possibility of energy being delivered multiple times at the same location on the skin, which can lead to too much energy in that location, cannot achieve the desired therapeutic effect, and also has the side effect of scalding the skin.

In this embodiment, the working process of the control method for outputting the ultrasound energy is as follows: detecting the moving speed of the ultrasound treatment applicator, and controlling the ultrasound energy output by the transducer according to the detected moving speed. There is a control logic for controlling the output of ultrasound energy in the preset speed interval corresponding to the detected moving speed, which is configured to control the transducer to output ultrasound energy. When the detected speed is less than or equal to the first preset speed value, the transducer is controlled to stop outputting the ultrasound energy. This embodiment controls the output of ultrasound energy by setting the speed feedback for the output of ultrasound energy, and controls the transducer not to output ultrasound energy when it is detected that the detected speed is less than or equal to the first preset speed value, thereby avoiding the side effect of ultrasound treatment and achieving the expected therapeutic effect.

In the present application, by setting the speed feedback for ultrasound energy, there is a matching relationship between the moving speed of the ultrasound treatment applicator and the magnitude of the ultrasound energy output, and the moving speed of the ultrasound treatment applicator is divided into multiple preset speed intervals. The transducer controls the output of ultrasound energy according to the control logic of the preset speed range. The ultrasound energy output is set according to the moving speed of the ultrasound treatment applicator to achieve the expected therapeutic effect. When the detected moving speed is less than or equal to the first preset speed value, the ultrasound treatment transducer stops outputting the ultrasound energy to the subcutaneous tissue. During operation, the possibility of the ultrasound treatment applicator stopping on the skin surface is detected by detecting the moving speed of the ultrasound treatment applicator. When the speed detected by the speed measuring sensor is less than or equal to the first preset speed value, it is determined that the control method for outputting the ultrasound energy is sufficiently small or the control method for outputting the ultrasound energy is stopped; and the ultrasound treatment transducer stops outputting the ultrasound energy to the subcutaneous tissue, so as to avoid the possibility of multiple output of ultrasound energy at the same position of the skin, avoid the side effects of treatment, and achieve the expected therapeutic effect. The present application solves the problem that ultrasound treatment cannot achieve the expected therapeutic effect due to improper personal operation by setting such a control method for outputting the ultrasound energy.

Referring to FIG. 2 and FIG. 5, in an embodiment of the present application, the step S20 further includes the following steps:
When the detected moving speed is greater than the first preset speed value and smaller than the second preset speed value, controlling the ultrasound energy output by the transducer to increase according to the increase of the detected moving speed.

In this embodiment, when the detected moving speed is greater than the first preset speed value, it is determined that the control method for outputting the ultrasound energy passes the movement, and the ultrasound treatment transducer starts to output ultrasound energy. The moving speed and the ultrasound output form a closed-loop feedback, and the greater the detected moving speed is, the greater the ultrasound energy output by the ultrasound treatment transducer is. When the detected moving speed is sufficiently large, the ultrasound energy output by the ultrasound treatment transducer no longer increases as the detected moving speed increases.

Since the higher the moving speed is, the greater the output ultrasound energy is, and the better the corresponding therapeutic effect is, but each doctor's operating habits and handle moving speed are different, if the moving speed is higher, the ultrasound treatment transducer still outputs smaller ultrasound energy, which will affect the therapeutic effect of the control method for outputting the ultrasound energy. The closed-loop feedback is formed by moving speed and ultrasound output, so as to avoid the influence of operating habits on the therapeutic effect.

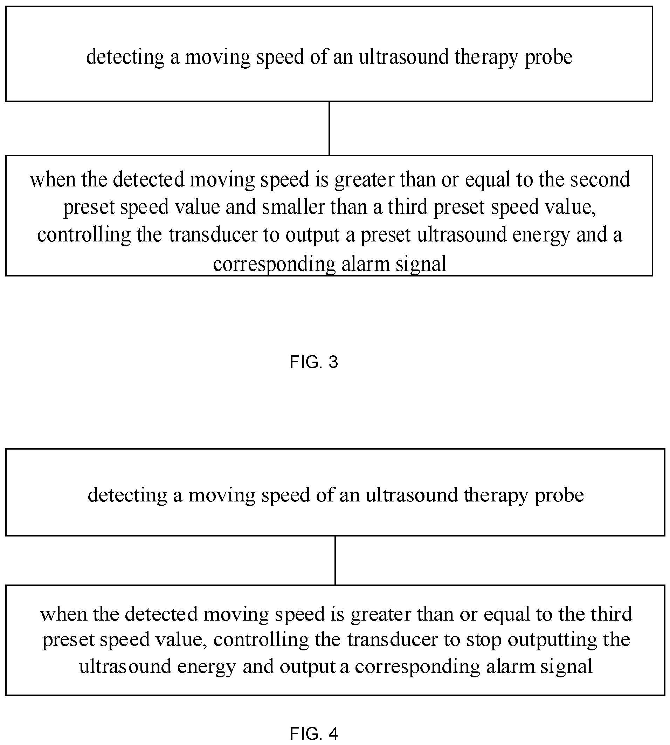

Referring to FIG. 3 and FIG. 5, in an embodiment of the present application, the step S20 further includes the following steps:
When the detected moving speed is greater than or equal to the second preset speed value and smaller than the third preset speed value, controlling the transducer to output the second preset ultrasound energy and the corresponding alarm signal.

In this embodiment, the ultrasound energy output by the ultrasound treatment transducer has a maximum value. During the working process of the control method for outputting the ultrasound energy, the ultrasound energy output by the ultrasound treatment transducer cannot exceed this maximum value. When the moving speed is large enough, the ultrasound energy output by the ultrasound treatment transducer no longer increases with the increase of the detected moving speed, but outputs a preset ultrasound energy value and a corresponding alarm signal.

In the process of practical application, the energy that can be configured by the ultrasound treatment transducer is limited. The ultrasound energy output by the ultrasound treatment transducer cannot exceed the upper limit of the configured energy. Excessive energy output by the ultrasound treatment transducer will also affect the load on its own work.

Referring to FIG. 4 and FIG. 5, in an embodiment of the present application, the step S20 further includes the following steps:
When the detected moving speed is greater than or equal to the third preset speed value, controlling the transducer to stop outputting the ultrasound energy and output the corresponding alarm signal.

In this embodiment, excessive moving speed cannot be applied to ultrasound treatment, and the expected therapeutic effect cannot be achieved. When the detected moving speed is greater than or equal to the third preset speed value, the ultrasound treatment transducer stops outputting the ultrasound energy. When the excessive moving speed is detected, it can be judged that the operator makes an operation error. At this time, the alarm will give an alarm, indicating that the moving speed of the treatment applicator is too high.

It can be understood that there is a corresponding relationship between the ultrasound energy output by the ultrasound treatment transducer and the moving speed of the control method for outputting the ultrasound energy.

When the detected moving speed is small, it is determined that the control method for outputting the ultrasound energy is small enough or the control method for outputting the ultrasound energy stops moving, and the ultrasound treatment transducer stops outputting the ultrasound energy to the subcutaneous tissue, so as to avoid the possibility of multiple output of ultrasound energy at the same position of the skin and reduce the risk of scalding the skin.

When the detected moving speed begins to increase, the ultrasound treatment transducer starts to output ultrasound energy to the subcutaneous tissue; and as the output ultrasound energy increases with the increase of the moving speed, the greater the output ultrasound energy, the better the corresponding therapeutic effect, so as to avoid the influence of operating habits on the therapeutic effect.

When the detected moving speed increases to a large value, the ultrasound energy output by the ultrasound treatment transducer no longer increases with the increase of moving speed, but outputs a preset ultrasound energy value and outputs a corresponding alarm signal to indicate that the moving speed is relatively high at this time and needs to be adjusted.

When the detected moving speed is extremely high, the output of the ultrasound energy output by the ultrasound treatment transducer is stopped. At this time, it is determined that the operator makes a wrong operation, and a corresponding alarm signal is sent to remind the operator of the wrong operation.

During operation, the ultrasound treatment transducer outputs ultrasound energy according to the moving speed detected by the speed measuring sensor. The moving speed in different intervals, the output energy of the ultrasound treatment transducer is different, so that the ultrasound treatment achieves the expected therapeutic effect.

Referring to FIG. 5, in an embodiment of the present application, when the detected moving speed is greater than the first preset speed value and smaller than the second preset speed value, the control method further includes the following steps:
Controlling the ultrasound energy output by the transducer to increase linearly according to the increase of the detected moving speed, that is, the growth rate of the output ultrasound energy with respect to the moving speed remains unchanged.

Referring to FIG. 6, in an embodiment of the present application, the step S20 further includes the following steps:
When the detected moving speed is greater than the first preset speed value and smaller than the second preset speed value, controlling the transducer to output the first preset ultrasound energy.

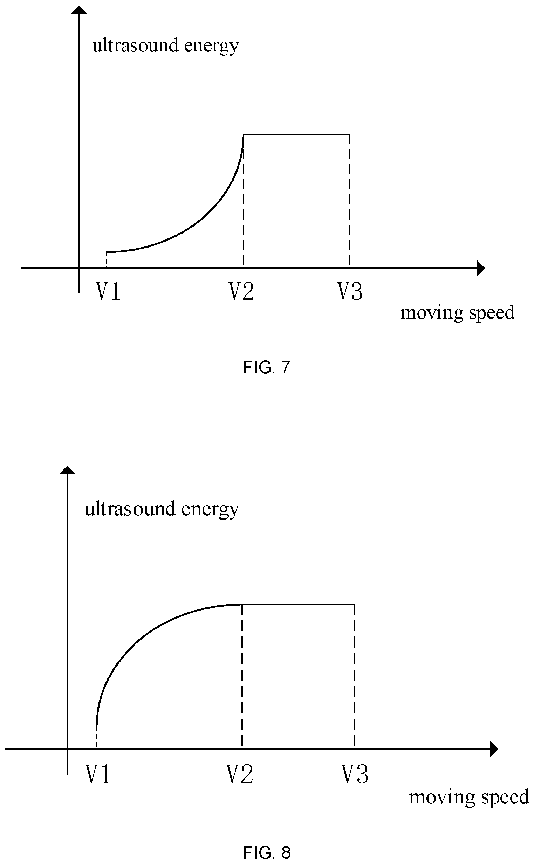

Referring to FIG. 7, in an embodiment of the present application, when the detected moving speed is greater than the first preset speed value and smaller than the second preset speed value, the ultrasound energy output by the transducer is controlled to increase exponentially according to the increase of the detected moving speed, that is, the growth rate of the output ultrasound energy with respect to the moving speed increases according to the increase of the detected moving speed.

Referring to FIG. 8, when the detected moving speed is greater than the first preset speed value and less than the second preset speed value, the ultrasound energy output by the transducer is controlled to increase logarithmically according to the increase of the detected moving speed, that is, the growth rate of the output ultrasound energy with respect to the moving speed decreases according to the increase of the detected moving speed.

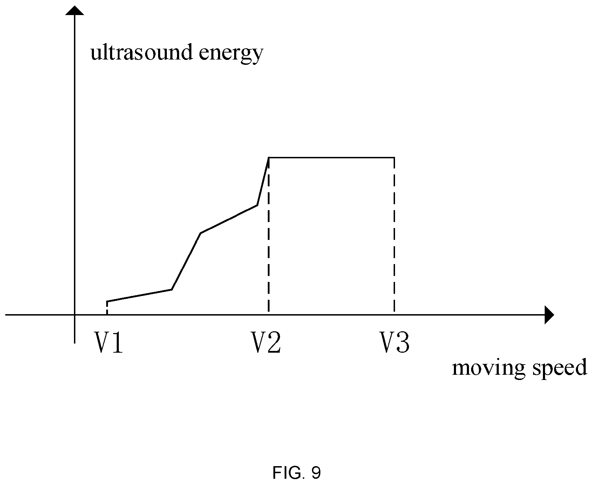

Referring to FIG. 9, when the detected moving speed is greater than the first preset speed value and less than the second preset speed value, the ultrasound energy output by the transducer is controlled to increase linearly in segments according to the increase of the detected moving speed; the following steps are specifically included:
detecting a preset speed interval divided from the moving speed greater than the first preset speed value and smaller than the second preset speed value;
determining the growth rate of the output ultrasound energy with respect to the moving speed according to the detected preset speed interval;
controlling the transducer to adjust the output ultrasound energy according to the determined growth rate and the change of the moving speed.

In this embodiment, the interval between the first preset speed value and the second preset speed value is divided into a plurality of growth intervals. Each of the multiple growth intervals corresponds to a change slope of the ultrasound output energy with respect to the moving speed, and the change of the output energy of the transducer is controlled according to the detected change of the moving speed and the determined change slope. The larger the slope of the change, the faster the output energy of the transducer changes according to the moving speed, which is a fast growth range; the smaller the slope of the change, the slower the output energy of the transducer changes according to the moving speed, which is a slow growth range.

In an embodiment of the present application, the following steps are also included before the step S10:
Detecting whether the surface of the ultrasound treatment applicator is in sufficient contact with the skin;
When it is detected that the surface of the ultrasound treatment applicator is in sufficient contact with the skin, performing the step S10, or, when it is detected that the surface of the ultrasound treatment applicator is in insufficient contact with the skin, outputting a corresponding alarm signal.

In this embodiment, before controlling the output of ultrasound energy according to the detected speed, it is firstly detected whether the surface of the ultrasound treatment applicator is in sufficient contact with the skin; if not, the step of speed feedback is no longer performed. Since the surface of the ultrasound treatment applicator is not fully in contact with the skin, the ultrasound energy output by the ultrasound treatment transducer may cause skin burns, the step of speed feedback is not performed, and a corresponding alarm signal is output to remind the operator.

In an embodiment of the present application, the step S20 further includes the following steps:
detecting the temperature of the skin surface;
when it is detected that the temperature of the surface of the ultrasound treatment applicator is higher than the preset temperature value, controlling the transducer to stop outputting the ultrasound energy; or, when the temperature of the surface of the ultrasound treatment applicator is detected lower than the preset temperature value, outputting an operation guide signal, and controlling the transducer to continue outputting the ultrasound energy. **In** this embodiment, the temperature sensor is configured to detect the temperature of the skin surface. When the temperature of the skin surface is too high, the ultrasound treatment transducer stops outputting the ultrasound energy to the subcutaneous tissue.

In this embodiment, when the ultrasound treatment transducer outputs ultrasound energy to the subcutaneous tissue, the temperature of the skin surface will also increase accordingly. When the detected temperature value is lower than the preset temperature value, it is determined that the temperature of the skin surface is still in a safe temperature area at this time, and a guide signal is output to remind the operator to continue the ultrasound treatment operation on the area.

When the detected temperature value is higher than the preset temperature value, it is determined that the temperature of the skin surface is too high, and the ultrasound treatment transducer stops outputting the ultrasound energy to the subcutaneous tissue, so as to avoid scalding the skin. And the corresponding alarm signal is output to remind the operator.

In an embodiment of the present application, the ultrasound output energy of the transducer is controlled by controlling the ultrasound output power and/or the ultrasound pulse output repetition frequency.

In this embodiment, the magnitude of the output ultrasound energy is specifically controlled by controlling the magnitude of the ultrasound output power and/or the magnitude of the ultrasound pulse output repetition frequency. The ultrasound energy output by the ultrasound treatment transducer is realized by controlling the ultrasound output power and the ultrasound pulse output repetition frequency. The ultrasound pulse output repetition frequency is the number of times the ultrasound treatment transducer outputs within a period of time, and the ultrasound output power is the power of a single output of the ultrasound treatment transducer.

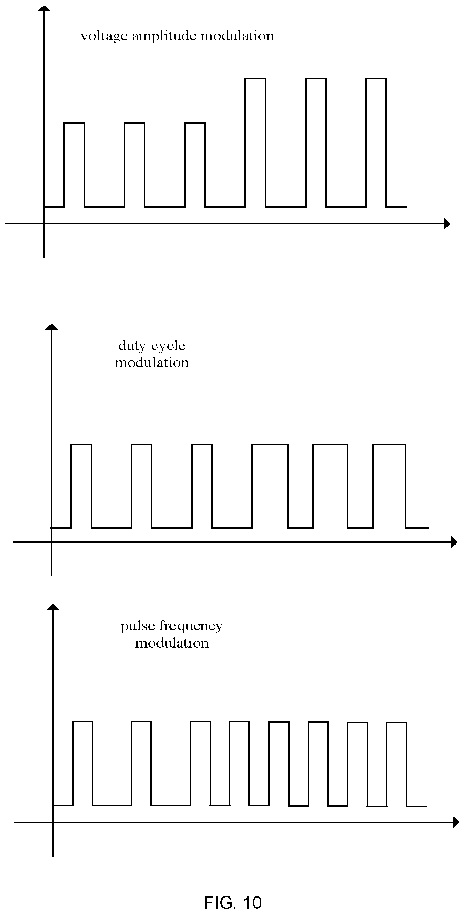

Referring to FIG. 10, in an embodiment of the present application, the time and voltage amplitude of the output pulse of the ultrasound unit are controlled to control the ultrasound output power of the transducer.

In this embodiment, the ultrasound output power of the transducer is realized by modulating its output pulse, specifically controlled by modulating the time and amplitude of the output pulse of the ultrasound unit. The longer the time for the ultrasound unit to output the pulse, the longer the corresponding time for the ultrasound unit to output the pulse, and the greater the amplitude of the output pulse, the longer the corresponding time for the ultrasound unit to output the pulse.

Referring to FIG. 10, in an embodiment of the present application, the frequency of the output pulse of the ultrasound unit and/or the duty cycle of the output pulse of the ultrasound unit are controlled to control the time of the output pulse of the ultrasound unit.

By controlling the frequency of the output pulse of the ultrasound unit and/or the duty cycle of the output pulse of the ultrasound unit to control the time of the output pulse of the ultrasound unit. The greater the frequency of the output pulse of the ultrasound unit is, the longer the time of the output pulse of the ultrasound unit is; and the larger the duty cycle of the output pulse of the ultrasound unit is, the longer the time for the output pulse of the ultrasound unit is.

The present application provides a control host.

In an embodiment of the present application, the control host includes a memory and a processor, and a control program for outputting the ultrasound energy is stored in the memory, and when the control program for outputting the ultrasound energy is performed by the processor, the steps of the control method for outputting the ultrasound energy described above are realized.

The treatment interface performs the steps of the control method including the above-mentioned ultrasound energy. The specific working steps of the control host refer to the above-mentioned embodiments. Since the treatment interface of the present application adopts all the technical solutions of all the above-mentioned embodiments, it has at least all above-mentioned beneficial effects brought by the technical solutions of the embodiments and will not be repeated here.

The present application provides an ultrasound treatment system.

In an embodiment of the present application, the ultrasound treatment system includes:
An ultrasound treatment applicator, provided with a transducer inside;
An ultrasound handle unit, on which the ultrasound treatment applicator is mounted, is configured to output ultrasound energy when sliding on the skin surface;
A sensor assembly, arranged on the ultrasound treatment applicator or the ultrasound handle unit, for detecting the moving speed of the ultrasound treatment applicator;
An alarm configured for alarming according to the detected moving speed;
The control host as mentioned above is connected with the ultrasound handle unit and is configured to control the operation of the ultrasound treatment applicator.

The ultrasound handle unit is a treatment handle. During treatment, the position of the output of ultrasound energy is controlled by moving the treatment handle and the control method for outputting the ultrasound energy, so as to achieve the therapeutic effect.

The treatment handle is moved by the operator during treatment to keep the ultrasound window sliding close to the skin, and the ultrasound energy is output in pulse form. By continuously moving the treatment handle, the position of the ultrasound energy output is controlled to achieve the therapeutic effect. The ultrasound treatment transducer installed on the ultrasound treatment applicator outputs configured ultrasound energy, which is manifested as a thermal effect on the tissue, and the heat diffuses from the focal point to its surroundings to form a hot zone/heat diffusion zone.

The sensor assembly arranged on the ultrasound treatment applicator or the ultrasound handle unit includes a speed sensor, which is configured to detect the moving speed of the ultrasound treatment applicator; and when the detected moving speed is greater than or equal to the third preset speed value, it is determined that the moving speed is too high and the operation is not normal at this time, and the alarm alarms to remind the operator.

In an embodiment of the present application, the sensor assembly is also configured to detect the temperature of the skin surface and whether the skin surface is in sufficient contact with the surface of the ultrasound treatment applicator, and the alarm is also configured to alarm when the sensor assembly detects that the temperature of the skin surface is greater than a preset temperature value, or when the sensor assembly detects that the skin surface is in sufficient contact with the surface of the ultrasound treatment applicator.

In this embodiment, the sensor assembly also includes a contact sensor, and the contact sensor detects whether the treatment applicator is fully attached to the skin. If not, the control host does not perform the working steps of speed feedback, and the alarm gives an alarm.

The sensor assembly also includes a temperature sensor, and when the temperature sensor detects that the temperature of the skin surface is higher than a preset temperature value, it stops outputting the ultrasound energy and sends an alarm through an alarm to remind the operator.

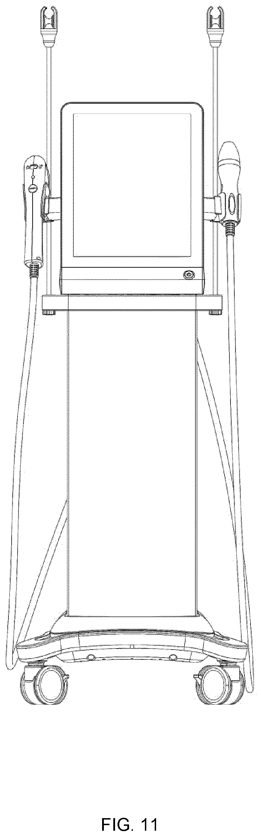

Referring to FIG. 11, in an embodiment of the present application, the control host further includes a system control unit and an ultrasound drive unit. The system control unit configures the working parameters of the ultrasound treatment applicator, and the ultrasound drive unit is configured to drive the ultrasound treatment applicator to work based on the working parameters configured by the control unit;
The system control unit configures the ultrasound output power and the ultrasound pulse output repetition frequency, and the ultrasound drive unit is used to drive the ultrasound treatment applicator to work according to the ultrasound output power and the ultrasound pulse output repetition frequency configured by the system control unit.

In this embodiment, the ultrasound energy adopts the dot-type pulse output mode, and each ultrasound energy hits a point to form an energy focus point in the tissue in turn. The adjustment of the ultrasound energy output includes the following two aspects.

The output frequency is controlled and adjusted, that is, the time interval between two adjacent ultrasound energy is changed, then the number of focus points shot per unit time is changed; when the output frequency increases, the ultrasound energy of each shot remains unchanged; and the number of shots shot per unit time increases, the output ultrasound energy per unit time increases.

The ultrasound power is controlled and adjusted, that is, the amplitude or pulse width of the voltage waveform of the ultrasound power supply is changed, thus changing the ultrasound energy of each shot; when the single shot of the ultrasound energy increases, the number of shots per unit time remains unchanged, and the output ultrasound energy per unit time increases.

In an embodiment of the present application, the system control unit is further configured to configure the time and/or voltage amplitude of the output pulse of the ultrasound unit to configure the ultrasound output power; and the ultrasound drive unit is configured to drive the ultrasound treatment applicator to work according to the ultrasound output power and the ultrasound pulse output repetition frequency configured by the system control unit.

In this embodiment, the system control unit is specifically used to modulate the output pulse of the transducer, and configure the ultrasound output power by controlling the time and/or voltage amplitude of the output pulse of the ultrasound unit of the transducer to drive the ultrasound treatment applicator to work.

The treatment handle is moved by the operator during treatment to keep the ultrasound window sliding close to the skin, and the ultrasound energy is output in pulse form. By continuously moving the treatment handle, the position of the ultrasound energy output is controlled to achieve the therapeutic effect. The ultrasound treatment transducer installed on the ultrasound treatment applicator outputs focused ultrasound energy, which is manifested as a thermal effect on the tissue, and the heat diffuses from the focused point to its surroundings to form a hot zone/heat diffusion zone.

The thermal diffusion zone formed by the ultrasound energy output by the treatment handle in a static state is small, and the temperature in the central area is relatively high; the thermal diffusion zone formed by the ultrasound energy output during the moving process of the treatment handle is relatively large, and the temperature of the central area of the thermal diffusion zone is lower than the center temperature of the thermal diffusion zone formed by the handle in the static state, which can effectively reduce the pain. Therefore, the energy distribution of the thermal diffusion zone formed by the handle in the moving state is more uniform and the experience is more comfortable.

In an embodiment of the present application, the working parameters of the ultrasound handle unit have specific ranges: output power is 1-30W, output frequency is 500K-15M, and the depth of action on subcutaneous tissue is 0.5-25mm.

In an embodiment of the present application, the specific range value of the working parameter of the treatment handle is: the repetition frequency is 2 Hz-50 Hz.

In an embodiment, the treatment handle is moved by the operator during treatment in a spiral circular trajectory centered at different locations within the treatment area.

In this embodiment, before the treatment handle is treated, the control method for outputting the ultrasound energy presses the skin to ensure that it is fully attached to the skin; during treatment, the treatment handle is controlled to move continuously, and the treatment applicator is slid continuously; the force is even when the treatment applicator slides; and the treatment applicator is moved in a spiral circle at the same point to realize the treatment operation in a small area and a small range to achieve the effect of treatment.

During treatment, circles can also be drawn centered on different positions in the treatment area. The centers of each circle are different and cross each other. Finally, the energy distribution in the entire treatment surface is relatively uniform, and the treatment handle can be moved to achieve the purpose of treatment. The energy output by the treatment is relatively uniform, and the temperature superposition effect is better, so as to achieve the therapeutic effect.

During treatment, the treatment handle can also be controlled to slide back and forth according to the Z-shaped trajectory in the treatment area. The force is uniform when sliding, and the ultrasound energy is continuously diffused by sliding back and forth, and finally the treatment energy is diffused to all treatment areas to achieve therapeutic effects.

The present application provides an ultrasound treatment device, which includes the above-mentioned ultrasound therapeutic system.

The ultrasound treatment device includes the ultrasound therapeutic system as described above. The specific structure of the ultrasound therapeutic system refers to the above-mentioned embodiments. Since the ultrasound treatment device of the present application adopts all the technical solutions of all the above-mentioned embodiments, it at least has all the beneficial effects brought by the technical solutions of the above-mentioned embodiments, and will not be repeated here.

The above are only some embodiments of the present application, and are not intended to limit the scope of the present application. Under the inventive concept of the present application, the equivalent structural transformations made by using the description of the application and the contents of the accompanying drawings, or direct/indirect applications in other relevant technical fields are included in the scope of the present application.

## Claims

1. A control method for outputting ultrasound energy, comprising:
step S10, detecting a moving speed of an ultrasound treatment applicator; and
step S20, controlling a transducer to output the ultrasound energy according to the detected moving speed,
wherein in response to that the detected moving speed is less than or equal to a first preset speed value, the transducer is controlled to stop outputting the ultrasound energy.

2. The control method for outputting the ultrasound energy according to claim 1, wherein the step S20 further comprises the following step:
in response to that the detected moving speed is greater than the first preset speed value and smaller than a second preset speed value, controlling the ultrasound energy output by the transducer to increase according to an increase of the detected moving speed.

3. The control method for outputting the ultrasound energy according to claim 1, wherein the step S20 further comprises the following step:
in response to that the detected moving speed is greater than the first preset speed value and smaller than a second preset speed value, controlling the transducer to output a first preset ultrasound energy.

4. The control method for outputting the ultrasound energy according to any one of claim 2 to 3, wherein the step S20 further comprises the following step:
in response to that the detected moving speed is greater than or equal to the second preset speed value and smaller than a third preset speed value, controlling the transducer to output a second preset ultrasound energy.

5. The control method for outputting the ultrasound energy according to any one of claim 2 to 3, wherein the step S20 further comprises the following step:
in response to that the detected moving speed is greater than or equal to a third preset speed value, controlling the transducer to stop outputting the ultrasound energy and output a corresponding alarm signal.

6. The control method for outputting the ultrasound energy according to claim 2, wherein in response to that the detected moving speed is greater than the first preset speed value and smaller than the second preset speed value, the ultrasound energy output by the transducer is controlled to increase linearly according to the increase of the detected moving speed.

7. The control method for outputting the ultrasound energy according to claim 2, wherein in response to that the detected moving speed is greater than the first preset speed value and smaller than the second preset speed value, the ultrasound energy output by the transducer is controlled to increase exponentially according to the increase of the detected moving speed.

8. The control method for outputting the ultrasound energy according to claim 2, wherein in response to that the detected moving speed is greater than the first preset speed value and smaller than the second preset speed value, the ultrasound energy output by the transducer is controlled to increase logarithmically according to the increase of the detected moving speed.

9. The control method for outputting the ultrasound energy according to claim 2, wherein in response to that the detected moving speed is greater than the first preset speed value and smaller than the second preset speed value, controlling the ultrasound energy output by the transducer to increase linearly in segments according to the increase of the detected moving speed comprises the following steps:
detecting a preset speed interval divided from the moving speed greater than the first preset speed value and smaller than the second preset speed value;
determining a growth rate of the output ultrasound energy with respect to the moving speed according to the detected preset speed interval; and
controlling the transducer to adjust the output ultrasound energy according to the determined growth rate and the change of the moving speed.

10. The control method for outputting the ultrasound energy according to claim 1, wherein before the step S10, the control method further comprises the following steps:
detecting whether a surface of the ultrasound treatment applicator is in sufficient contact with a skin; and
in response to that the surface of the ultrasound treatment applicator is in sufficient contact with the skin, performing the step S10; or, in response to that the surface of the ultrasound treatment applicator is in insufficient contact with the skin, outputting a corresponding alarm signal.

11. The control method for outputting the ultrasound energy according to claim 10, wherein the step S20 further comprises the following steps:
detecting whether the surface of the ultrasound treatment applicator is in sufficient contact with the skin; and
in response to that the surface of the ultrasound treatment applicator is in insufficient contact with the skin, controlling the transducer to stop outputting the ultrasound energy and output the corresponding alarm signal; or, in response to that the surface of the ultrasound treatment applicator is in sufficient contact with the skin, performing the step S10.

12. The control method for outputting the ultrasound energy according to claim 1, wherein the step S20 further comprises the following steps:
detecting a temperature of a skin surface; and
in response to that the temperature on the surface of the ultrasound treatment applicator is higher than a preset temperature value, controlling the transducer to stop outputting the ultrasound energy and output a corresponding alarm signal; or, in response to that the temperature on the surface of the ultrasound treatment applicator is lower than the preset temperature value, outputting an operation guide signal, and controlling the transducer to continue to output the ultrasound energy.

13. The control method for outputting the ultrasound energy according to any one of claims 1 to 12, further comprising:
controlling an ultrasound output power and/or an ultrasound pulse output repetition frequency to control an ultrasound output energy of the transducer.

14. The control method for outputting the ultrasound energy according to claim 13, further comprising:
controlling a time and/or a voltage amplitude of an output pulse of an ultrasound unit to control the ultrasound output power of the transducer.

15. The control method for outputting the ultrasound energy according to claim 14, further comprising:
controlling a frequency of the output pulse of the ultrasound unit and/or a duty ratio of the output pulse of the ultrasound unit to control the time of the output pulse of the ultrasound unit.

16. A control host, comprising:
a memory; and
a processor,
wherein a control program for outputting ultrasound energy is stored in the memory, and when the control program for outputting the ultrasound energy is performed by the processor, the steps of the control method for outputting the ultrasound energy according to any one of claims 1 to 15 are realized.

17. An ultrasound treatment system, comprising:
an ultrasound treatment applicator provided with a transducer inside;
an ultrasound handle unit; wherein the ultrasound treatment applicator is mounted on the ultrasound handle unit, and the ultrasound handle unit is configured to output ultrasound energy when sliding on a skin surface;
a sensor assembly arranged on the ultrasound treatment applicator or the ultrasound handle unit and configured for detecting a moving speed of the ultrasound treatment applicator;
an alarm configured for alarming according to the detected moving speed; and
the control host according to claim 16; wherein the control host is connected with the ultrasound handle unit and configured to control the operation of the ultrasound treatment applicator.

18. The ultrasound treatment system according to claim 17, wherein the sensor assembly is also configured to detect the temperature of the skin surface and/or whether the skin surface is in sufficient contact with the surface of the ultrasound treatment applicator; and the alarm is also configured to detect the temperature of the skin surface and/or alarm based on whether the skin surface is in sufficient contact with the surface of the ultrasound treatment applicator.

19. The ultrasound treatment system according to claim 17, wherein the control host further comprises a system control unit and an ultrasound drive unit;
the system control unit configures working parameters of the ultrasound treatment applicator, and the ultrasound drive unit is configured to drive the ultrasound treatment applicator to work based on the operating parameters configured by the system control unit;
wherein the system control unit is configured to configure the ultrasound output power and the ultrasound pulse output repetition frequency, and the ultrasound drive unit is configured to drive the ultrasound treatment applicator to work according to the ultrasound output power and the ultrasound pulse output repetition frequency configured by the system control unit.

20. The ultrasound treatment system according to claim 19, wherein the system control unit is further configured to configure the time and/or the voltage amplitude of the output pulse of the ultrasound unit to configure the ultrasound output power; and the ultrasound drive unit is configured to drive the ultrasound treatment applicator to work based on the ultrasound output power and ultrasound pulse output repetition frequency configured by the system control unit.

21. A treatment device, comprising the ultrasound treatment system according to any one of claims 17 to 20.
